# EUROPÄISCHE PATENTSCHRIFT

(11) **EP 1 966 120 B1**
(45) Veröffentlichungstag und Bekanntmachung des Hinweises auf die Patenterteilung: **28.11.2012**
(21) Anmeldenummer: 06830685.1
(22) Anmeldetag: 18.12.2006
(51) Int. Cl.: C07C 205/24, C06B 25/04, C06C 9/00, C06B 41/00, C06C 7/00

(54) **SALZE DER STYPHNINSÄURE**
SALTS OF STYPHNIC ACID
SELS DE L'ACIDE STYPHNIQUE

(30) Priorität: 20.12.2005 DE 102005061324
(43) Veröffentlichungstag der Anmeldung: 10.09.2008
(73) Patentinhaber: RUAG Ammotec GmbH, 90765 Fürth (DE)
(72) Erfinder: BLEY, Ulrich, 90766 Fürth (DE); HAGEL, Rainer, 91058 Erlangen (DE); HOSCHENKO, Aleksej, 90765 Fürth (DE); LECHNER, Dr. Peter Simon, 90522 Oberasbach (DE)
(74) Vertreter: Uppena, Franz
(86) Internationale Anmeldenummer: PCT/EP2006/069846
(87) Internationale Veröffentlichungsnummer: WO 2007/071649

(56) Entgegenhaltungen:
- WO-A-99/48842
- DE-A1- 2 640 799
- DE-A1- 4 117 719
- DE-A1- 10 009 685
- ZAFAR, M. A.: "Initiation of explosive powders by a laser beam" DHAKA UNIVERSITY STUDIES, PART B: SCIENCE , 35(1), 15-22 CODEN: DUBSDX; ISSN: 0259-7365, 1987, XP008082753
- DATABASE CA [Online] CHEMICAL ABSTRACTS SERVICE, COLUMBUS, OHIO, US; SVETLOV, B. S. ET AL: "Effect of pressure on combustion of some salts of explosive acids" XP002447530 gefunden im STN Database accession no. 1968:51474 & TRUDY KAZANSKOGO KHIMIKO-TEKHNOLOGICHESKOGO INSTITUTA , NO. 53, 328-38 CODEN: TKKKAL; ISSN: 0371-8603, 1967,
- DATABASE CA [Online] CHEMICAL ABSTRACTS SERVICE, COLUMBUS, OHIO, US; ZHENG, HONG ET AL: "Synthesis, X-ray crystal structure and thermal decomposition mechanism of [RbHTNR].infin." XP002447531 gefunden im STN Database accession no. 2006:757125 & CHINESE JOURNAL OF CHEMISTRY , 24(7), 845-848 CODEN: CJOCEV; ISSN: 1001-604X, 2006,
- DATABASE CA [Online] CHEMICAL ABSTRACTS SERVICE, COLUMBUS, OHIO, US; ZHENG, HONG ET AL: "Synthesis, crystal structure, and thermal behavior of [Cs2 (TNR).bul.2(H2O)]n" XP002447532 gefunden im STN Database accession no. 2006:477586 & HANNENG CAILIAO , 14(1), 80 CODEN: HACAFQ; ISSN: 1006-9941, 2006,

## Beschreibung

Gegenstand der Erfindung sind Salze der Styphninsäure, Verfahren zu deren Herstellung und deren Verwendung.

Herkömmliche Anzündstoffe, die beispielsweise in Fahrzeugsicherheitssystemen eingesetzt werden, haben den Nachteil, dass sie wegen niedriger Zersetzungstemperaturen nicht im Motorraum von Kfz eingesetzt werden können. Im Motorraum eines Kfz werden Temperaturen von 140°C und mehr erreicht, was eine Zersetzungstemperatur eines Anzündstoffes von über 300°C erfordert. Kaliumdinitrobenzofuroxanat beispielsweise hat eine Zersetzungstemperatur von ca. 220°C und ist daher für diesen Zweck ungeeignet.

Aus dem Dokument WO 99/48842 A sind Initialexplosivstoffe zum Anzünden von Boostern oder Treibladungen bzw. zum Erzeugen von Druckgas bekannt, die als Initialexplosivstoff ein Salz des Trinitroresorcins wie das Kallum- und/oder Strontiumsalz enthalten, wobei jedoch keinerlei Hinweise auf die Bedeutung der Zersetzungstemperatur des eingesetzten Initialexplosivstoffes gegeben werden.

Aufgabe der vorliegenden Erfindung war es, einen Stoff bereitzustellen, der als Anzündstoff geeignet ist und die Nachteile des Standes der Technik überwindet. Weitere Aufgaben bestanden darin, einen Anzündstoff bereitzustellen, der eine Zersetzungstemperatur hat, die über 300°C liegt, der schwermetallfrei ist, der sich sowohl für mechanische als auch für elektrische Anzündsysteme eignet, der beispielsweise in Fahrzeugsicherheitssysteme, Munition und Treibkartuschen für Bolzensetzgeräte eingesetzt werden kann und/oder dessen Herstellung durch einfache technische Verfahren möglich ist.

Erfindungsgemäß werden diese Aufgaben überraschenderweise durch die Merkmale der Ansprüche 1, 4. bis 6 und 7 bis 11 gelöst. Vorzugsweise Ausgestaltungen finden sich in den Unteransprüchen.

Überraschenderweise wurde gefunden, dass diese Aufgaben durch das Mischsalz Kalium-Calcium-Styphnat und basisches Calciumstyphnat sowie basisches Kalium-Calcium-Styphnat der Styphninsäure (2,4,6-Trinitro-1,3-Dihydroxybenzol) nachfolgend kurz Styphnate genannt - deren Herstellung und erfindungsgemäßer Verwendung gelöst werden.

Die Herstellung des erfindungsgemäßen Anzündstoffes gelingt ausgehend von einer wässrigen Magnesiumstyphnatlösung:
- das basische Calciumstyphnat bildet sich bei der Zugabe von Alkalihydroxiden zu einer Calciumstyphnat-Suspension;
- im Falle der Herstellung von Kalium-Calcium-Styphnat durch die Fällung aus Kaliumstyphnatlösung unter stöchiometrischer Zugabe von Calciumnitrat; das basische Kalium-Calcium-Styphnat bildet sich bei der Zugabe von Alkalihydroxiden zu einer Kalium-Calcium-Styphnat-Suspension.

Diese Styphnate können erfindungsgemäß als Anzündstoff sowohl einzeln als auch in Mischung untereinander und/oder ggf. in Mischung mit den üblichen Zusatzstoffen wie beispielsweise Oxidationsmittel, Reduktionsmittel, Sensibilisatoren, Bindemittel, energiereiche Zuschläge sowie Abbrandmoderatoren und Verarbeitungshilfen eingesetzt werden.

Als Zusatzstoffe beim Einsatz der erfindungsgemäßen Stoffe als Anzündstoff, beispielsweise in Anzündsystemen, können erfindungsgemäß verwendet werden:
1. Oxidationsmittel (einzeln oder in Mischungen):
   Nitrate der Alkali- oder Erdalkalimetalle oder des Ammoniums wie Natriumnitrat oder Kaliumnitrat, Perchlorate der Alkali- oder Erdalkalimetalle oder des Ammoniums, Peroxide der Erdalkalimetalle oder des Zinks, bevorzugt Zinkperoxid.
2. Reduktionsmittel (einzeln oder in Mischungen):
   Aluminium, Titan, Titanhydrid, Bor, Borhydrid, Zirkon, Zirkonhydrid, Silicium, Graphit, Aktivkohle, Ruß, bevorzugt Titan.
3. Sensibilisatoren (einzeln oder in Mischungen):
   Tetrazen, Kaliumdinitrobenzofuroxanat, Diazodinitrophenol.
4. Bindemittel (einzeln oder in Mischungen):
   Adhesin, Cellulose sowie deren Derivate, Polyvinylbutyrale, Polynitropolyphenylen, Polynitrophenylether, Plexigum, Polyvinylacetat und Copolymere, bevorzugt Adhesin.
6. Energiereiche Zuschläge (einzeln oder in Mischungen):
   Hexogen, Oktogen, Nitropenta und Nitrocellulose.
7. Abbrandmoderatoren und Verarbeitungshilfen (einzeln oder in Mischungen):
   Nitrocellulose-Kugelpulver, Acetonylacetate, Salicylate, Silikate, Kieselgele, Bornitrid, bevorzugt Nitrocellulose-Kugelpulver.

Bei Einsatz der erfindungsgemäßen Stoffe als Anzündstoff zeichnet sich dieser durch Schwermetallfreiheit, hohe thermische Stabilität und im Falle von basischem Calciumstyphnat und Kalium-Calcium-Styphnat sowie basischem Kalium-Calcium-Styphnat durch den Gehalt an Calcium aus, welches durch Bildung von Calciumcarbonat in den Verbrennungsrückständen aufgrund der günstigen tribologischen Eigenschaften des Calciumcarbonats für Waffensysteme vorteilhaft ist. Die Zersetzungstemperaturen der erfindungsgemäßen Styphnate liegen bei ca. 335°C. Der Anzündstoff lässt sich sowohl mechanisch als auch elektrisch anzünden.

Gegenstand der Erfindung ist im Einzelnen:
- ein Alkali- und/oder Erdalkalisalz der Styphninsäure als Anzündstoff, wobei es sich um das Mischsalz Kalium-Calcium-Styphnat, basisches Calciumstyphnat und basisches Kalium-Calcium-Styphnat handelt;
- ein Kalium-Calcium-Styphnat, das 10 bis 15, bevorzugt 12 bis 14 Gew.-% Kalium und 5 bis 10, bevorzugt 6 bis 8 Gew.-% Calcium enthält;
- ein basisches Kalium-Calcium-Styphnat, das 10 bis 15, bevorzugt 11 bis 13 Gew.-% Kalium und 10 bis 15, bevorzugt 11 bis 13 Gew.-% Calcium enthält;
- ein Verfahren zur Herstellung von basischem Calciumstyphnat durch Zugabe von Alkalihydroxiden zu einer Calciumstyphnat-Suspension;
- ein Verfahren zur Herstellung von Kalium-Calcium-Styphnat durch die Fällung aus Kaliumstyphnatlösung durch Zugabe von Calciumnitrat, bevorzugt durch stöchiometrische Zugabe von Calciumnitrat;
- ein Verfahren zur Herstellung von basischem Kalium-Calcium-Styphnat durch Zugabe von Alkalihydroxiden zu einer Kalium-Calcium-Styphnat-Suspension;
- die Verwendung von Kalium-Calcium-Styphnat, basischem Calciumstyphnat oder basischem Kolium-Calcium-Styphnat als Anzündstoffe;
- die Verwendung von Kalium-Calcium-Styphnat, basischem Calciumstyphnat oder basischem Kalium-Calcium-Styphnat als Bestandteil von Anzündsätzen, bevorzugt als Anzündstoff in Anzündsätzen;
- die Verwendung von Kalium-Calcium-Styphnat, basischem Calciumstyphnat oder basischem Kolium-Calcium-Styphnat in Anzündsystemen für Fahrzeugsicherheitssysteme, Munition und/oder Treibkartuschen für Bolzensetzgeräte;
- die Verwendung von Kalium-Calcium-Styphnat, basischem Calciumstyphnat oder basischem Kolium-Calcium-Styphnat in Anzündsystemen für Fahrzeugsicherheitssystemen, bevorzugt in solchen, die im Motorraum eines Kfz eingesetzt werden;
- die Verwendung von Kalium-Calcium-Styphnat, basischem Calciumstyphnat oder basischem Kolium-Calcium-Styphnat in Anzündsystemen, die elektrisch gezündet werden.

Nachfolgend ist die Erfindung beispielhaft erläutert, ohne die Erfindung auf die Beispiele einzuschränken:

### Beispiel 1 : Kalium-Calcium-Styphnat:

Basisches Kalium-Calcium-Styphnat, hergestellt durch die Fällung aus Kaliumstyphnatlösung unter stöchiometrischer Zugabe von Calciumnitrat, wurde mit Atomabsorptionsspektroskopie untersucht. Es wurden folgende Anteile von Kalium und Calcium gefunden:
- Kalium:: ca. 13Gew.-%
- Calcium:: ca. 7 Ges.-%

### Beispiel 2: basisches Kalium-Calcium-Styphnat:

Kalium-Calcium-Styphnat, hergestellt durch die Zugabe von Alkalihydroxiden zu einer Kalium-Calcium-Styphnat-Suspension, wurde mit Atomabsorptionsspektroskopie untersucht. Es wurden folgende Anteile von Kalium und Calcium gefunden:
- Kalium:: ca. 11,5 Gew.-%.
- Calcium:: ca. 12 Gew.-%

In Tabelle 1 sind die Zersetzungstemperaturen, Reib- und Schlagempfindlichkeiten der Substanzen dargestellt. Die Messung der Reib- und Schlagempfindlichkeiten erfolgte nach Methoden der Bundesanstalt für Materialforschung (BAM), während die Messung der Zersetzungstemperaturen mit der Thermogravimetrie-Analyse (Fa. Mettler) bei einer Aufheizrate von 10°C pro Minute erfolgte.

**Tabelle 1:**

| | Kalium-Calcium-Styphnat | basisches Kalium-Calcium-Styphnat | basisches Calcium-styphnat |
|---|---|---|---|
| Reibempfindlichkeit in N | 9 | 9 | 9 |
| Schlagempfindlichkeit in J | 3 | 3 | 4 |
| Zersetzungstemperatur in °C | 345 | 340 | 335 |

## Patentansprüche

1. **Salz der Styphninsäure, dadurch gekennzeichnet, dass es das Mischsalz Kalium-Calcium-Styphnat, das basische Calciumstyphnat oder basisches Kalium-Calcium-Styphnat ist.**

2. Kalium-Calcium-Styphnat nach Anspruch 1, **dadurch gekennzeichnet, dass** es 10 bis 15 Gew.-% Kalium und 5 bis 10 Gew.-% Calcium enthält.

3. Basisches Kalium-Calcium-Styphnat nach Anspruch 1, **dadurch gekennzeichnet, dass** es 10 bis 15 Gew.-% Kalium und 10 bis 15 Gew.-% Calcium enthält.

4. Verfahren zur Herstellung von basischem Calciumstyphnat gemäß Anspruch 1, **dadurch gekennzeichnet, dass** zu einer Calciumstyphnat-Suspension Alkalihydroxid zugegeben wird.

5. Verfahren zur Herstellung von Kalium-Calcium-Styphnat gemäß Anspruch 1 oder 2, **dadurch gekennzeichnet, dass** einer Kaliumstyphnatlösung Calciumnitrat zugegeben wird.

6. Verfahren zur Herstellung von basischem Kalium-Calcium-Styphnat gemäß Anspruch 1 oder 3, **dadurch gekennzeichnet, dass** zu einer Kalium-Calcium-Styphnat-Suspension Alkalihydroxid zugegeben wird.

7. Verwendung von einem oder mehreren Salzen der Styphninsäure nach mindestens einem der Ansprüche 1 bis 3 als Anzündstoff.

8. Verwendung von einem oder mehreren Salzen der Styphninsäure nach mindestens einem der Ansprüche 1 bis 3 als Bestandteil von Anzündsätzen.

9. Verwendung von einem oder mehreren Salzen der Styphninsäure nach mindestens einem der Ansprüche 1 bis 3 in Anzündsystemen für Fahrzeugsicherheitssysteme, Munition und/oder Treibkartuschen für Bolzensetzgeräte.

10. Verwendung von einem oder mehreren Salzen der Styphninsäure nach mindestens einem der Ansprüche 1 bis 3 in Anzündsystemen von Fahrzeugsicherheitssystemen,

11. Verwendung von einem oder mehreren Salzen der Styphninsäure nach mindestens einem der 1 bis 3 in Anzündsystemen, die elektrisch gezündet werden.

## Claims

1. A salt of styphnic acid, **characterised in that** it is the mixed salt potassium-calcium styphnate, the basic calcium styphnate or basic potassium-calcium styphnate.

2. Potassium-calcium styphnate according to claim 1, **characterised in that** it contains 10 to 15 wt.% potassium and 5 to 10 wt.% calcium.

3. Basic potassium-calcium styphnate according to claim 1, **characterised in that** it contains 10 to 15 wt.% potassium and 10 to 15 wt.% calcium.

4. A process for the preparation of basic calcium styphnate according to claim 1, **characterised in that** alkali hydroxide is added to a calcium styphnate suspension.

5. A process for the preparation of potassium-calcium styphnate according to claim 1 or 2, **characterised in that** calcium nitrate is added to a potassium styphnate solution.

6. A process for the preparation of basic potassium-calcium styphnate according to claim 1 or 3, **characterised in that** alkali hydroxide is added to a potassium-calcium styphnate suspension.

7. The use of one salt or a plurality of salts of styphnic acid according to at least one of claims 1 to 3 as a primer substance.

8. The use of one salt or a plurality of salts of styphnic acid according to at least one of claims 1 to 3 as a component of primer compositions.

9. The use of one salt or a plurality of salts of styphnic acid according to at least one of claims 1 to 3 in ignition systems for vehicle safety systems, ammunition and/or propellant cartridges for powder actuated tools.

10. The use of one salt or a plurality of salts of styphnic acid according to at least one of claims 1 to 3 in ignition systems of vehicle safety systems.

11. The use of one salt or a plurality of salts of styphnic acid according to at least one of claims 1 to 3 in ignition systems which are ignited electrically.

## Revendications

1. Sel de l'acide styphnique, **caractérisé en ce qu'**il s'agit du sel mixte styphnate de potassium et de calcium, du styphnate de calcium basique ou du styphnate de potassium et de calcium basique.

2. Styphnate de potassium et de calcium, conforme à la revendication 1, **caractérisé en ce qu'**il contient de 10 à 15 % en poids de potassium et de 5 à 10 % en poids de calcium.

3. Styphnate de potassium et de calcium basique, conforme à la revendication 1, **caractérisé en ce qu'**il contient de 10 à 15 % en poids de potassium et de 10 à 15 % en poids de calcium.

4. Procédé de préparation du styphnate de calcium basique conforme à la revendication 1, **caractérisé en ce que** l'on ajoute un hydroxyde alcalin à une suspension de styphnate de calcium.

5. Procédé de préparation du styphnate de potassium et de calcium conforme à la revendication 1 ou 2, **caractérisé en ce que** l'on ajoute du nitrate de calcium à une solution de styphnate de potassium.

6. Procédé de préparation du styphnate de potassium et de calcium basique conforme à la revendication 1 ou 3, **caractérisé en ce que** l'on ajoute un hydroxyde alcalin à une suspension de styphnate de potassium et de calcium.

7. Utilisation d'un sel ou de plusieurs sels de l'acide styphnique conforme(s) à au moins l'une des revendications 1 à 3, en tant que matériau d'amorce.

8. Utilisation d'un sel ou de plusieurs sels de l'acide styphnique conforme(s) à au moins l'une des revendications 1 à 3, en tant que constituants d'amorces.

9. Utilisation d'un sel ou de plusieurs sels de l'acide styphnique conforme(s) à au moins l'une des revendications 1 à 3, dans des systèmes d'amorçage pour systèmes de sécurité de véhicule, des munitions et/ou des cartouches explosives pour pistolets de scellement.

10. Utilisation d'un sel ou de plusieurs sels de l'acide styphnique conforme(s) à au moins l'une des revendications 1 à 3, dans des systèmes d'amorçage de systèmes de sécurité de véhicule.

11. Utilisation d'un sel ou de plusieurs sels de l'acide styphnique conforme(s) à au moins l'une des revendications 1 à 3, dans des systèmes d'amorçage à allumage électrique.
